# EUROPÄISCHE PATENTSCHRIFT

(11) **EP 3 958 951 B1**
(45) Veröffentlichungstag und Bekanntmachung des Hinweises auf die Patenterteilung: **04.06.2025**
(21) Anmeldenummer: 20714590.5
(22) Anmeldetag: 26.03.2020
(51) Int. Cl.: A61N 1/05, C08G 61/00, A61B 5/00

(54) **FLEXIBLE IMPLANTIERBARE ELEKTRODENANORDNUNG UND HERSTELLUNGSVERFAHREN**
FLEXIBLE IMPLANTABLE ELECTRODE ARRANGEMENT AND PRODUCTION METHOD
AGENCEMENT D'ÉLECTRODES IMPLANTABLE SOUPLE ET PROCÉDÉ DE FABRICATION

(30) Priorität: 26.04.2019 DE 102019205991
(43) Veröffentlichungstag der Anmeldung: 02.03.2022
(73) Patentinhaber: Albert-Ludwigs-Universität Freiburg, 79098 Freiburg (DE)
(72) Erfinder: STIEGLITZ, Thomas, 79110 Freiburg (DE); GUELI, Calogero, 79098 Freiburg (DE); VOMERO, Maria, 79098 Freiburg (DE); SHARMA, Swati, 76131 Karlsruhe (DE)
(74) Vertreter: Grünecker Patent- und Rechtsanwälte PartG mbB
(86) Internationale Anmeldenummer: PCT/EP2020/058480
(87) Internationale Veröffentlichungsnummer: WO 2020/216573

(56) Entgegenhaltungen:
- WO-A1-2019/046631
- CN-A- 108 744 268
- US-A1- 2016 073 920
- SURABHI NIMBALKAR ET AL: "Ultra-Capacitive Carbon Neural Probe Allows Simultaneous Long-Term Electrical Stimulations and High-Resolution Neurotransmitter Detection", SCIENTIFIC REPORTS, vol. 8, no. 1, 3 May 2018 (2018-05-03), XP055700408, DOI: 10.1038/s41598-018-25198-x
- MARIA VOMERO ET AL: "Glassy Carbon Electrocorticography Electrodes on Ultra-Thin and Finger-Like Polyimide Substrate: Performance Evaluation Based on Different Electrode Diameters", MATERIALS, vol. 11, no. 12, 1 December 2018 (2018-12-01), CH, pages 2486, XP055700401, ISSN: 1996-1944, DOI: 10.3390/ma11122486
- KASSEGNE SAM ET AL: "Electrical impedance, electrochemistry, mechanical stiffness, and hardness tunability in glassy carbon MEMS [mu]ECoG electr", MICROELECTRONIC ENGINEERING, vol. 133, 22 November 2014 (2014-11-22), pages 36 - 44, XP029127146, ISSN: 0167-9317, DOI: 10.1016/J.MEE.2014.11.013

## Beschreibung

Die vorliegende Erfindung bezieht sich auf flexible implantierbare Elektrodenanordnungen, z. B. Elektrodenarrays, und auf ein zugehöriges Herstellungsverfahren.

Jüngste Forschung und Entwicklung auf dem Gebiet der Neurotechnik ("Neural Engineering") führten zu einer Vielzahl von aktiven implantierbaren medizinischen Geräten (Active Implantable Medical Device, AIMD), die in einem weiten Anwendungsbereich einsetzbar sind. Diese bestehen üblicherweise aus einem Gehäuse, das eine Steuerelektronik und eine Batterie beinhaltet, implantierbaren Elektroden (oder Elektrodenarrays) und Kabeln für die elektrische Kontaktierung der Elektroden und der Elektronik. Die Elektroden werden für die elektrische Stimulierung von Zellen oder das Erfassen von physiologischen Signalen verwendet.

Damit dienen neuronale Elektroden als Schnittstelle zwischen dem biologischen und dem technischen System, wobei ihre Aufgabe im Wesentlichen in der Aufzeichnung und/oder der Anregung neuronaler Signale besteht. Wenn neuronale Elektroden in AIMD eingesetzt werden, spielen sie eine Schlüsselrolle bei der Wiederherstellung und Aufrechterhaltung von Körperfunktionen bei Patienten mit physischen Handicaps. Derartige Elektroden besitzen ein elektrisch leitfähiges Material für die Kontaktbereiche und die Verbindungsstellen sowie ein Substratmaterial, das die elektrisch leitfähigen Materialien isoliert. Wesentliche Voraussetzungen für den Erfolg implantierbarer Medizingeräte ist zum einen eine vorteilhafte Gewebe-Elektroden-Wechselwirkung und zum anderen eine ausreichende Biostabilität. Aus diesem Grund ist die mechanische Flexibilität der Elektrode ein essenzieller Aspekt beim Design von neuronalen Sonden, um eine strukturelle Biokompatibilität zu erzielen und dadurch die Fremdkörperreaktion zu verringern und die Lebensdauer des Implantats zu erhöhen.

Elektrisch leitfähige Carbonmaterialien erfüllen die Anforderungen bezüglich der Biostabilität ebenso wie bezüglich der Aufzeichnungs- und Stimulationsfähigkeiten, aber sie besitzen üblicherweise nicht die Fähigkeit, gebogenen Trajektorien zu folgen ohne zu brechen, da sie hart und spröde sind. Daher wird heutzutage Carbonmaterial nur an den Kontaktstellen der Elektrode innerhalb einer vergleichsweise kleinen Fläche verwendet, während die Leiterbahnen aus dünnen Metallfilmen hergestellt werden. Solche Elektroden sind beispielsweise in der Publikation S. Kassegne, "Electrical impedance, electrochemistry, mechanical stiffness, and hardness tunability in glassy carbon MEMS µECoG electrodes", Microelectronic Engineering, vol. 113, pp. 36-44, 2015, gezeigt. Teilweise werden auch Haftvermittler zwischen dem Carbonmaterial und dem Metall eingesetzt (siehe M. Vomero, "Incorporation of Silicon Carbide and Diamond-Like Carbon as Adhesion Promoters Improves In Vitro and In Vivo Stability of Thin-Film Glassy Carbon Electrocorticography Arrays", Advanced Biosystems, vol. 2, p. 170081, 2018).

Der Fachartikel SURABHI NIMBALKAR ET AL: "Ultra-Capacitive Carbon Neural Probe Allows Simultaneous Long-Term Electrical Stimulations and High-Resolution Neurotransmitter Detection", SCIENTIFIC REPORTS, Bd. 8, Nr. 1, 3. Mai 2018, offenbart neuronale Sonden auf Kohlenstoffbasis, die aus homogenen Mikroelektroden aus glasartigem Kohlenstoff (GC), Verbindungselementen und Bump-Pads bestehen. Diese Elektroden haben ein rein kapazitives Verhalten mit einer außergewöhnlich hohen Ladungsspeicherkapazität (CSC) und sind in der Lage, mehr als 3,5 Milliarden Zyklen von zweiphasigen Impulsen bei einer Ladungsdichte von 0,25 mC/cm² auszuhalten. Die Sonden ermöglichen sowohl die Aufzeichnung elektrischer Signale mit hohem SNR (>16) als auch eine bemerkenswert hochauflösende Echtzeit-Detektion von Neurotransmittern auf der gleichen Plattform. Die Sonden werden mittels einer zweiseitigen 2-Schritt-Strukturübertragungsmethode für GC-Strukturen hergestellt. Damit ermöglichen diese Sonden eine verlängerte elektrische Langzeitstimulation ohne Korrosion des Elektrodenmaterials. Die Querschnittscharakterisierung mittels FIB- und SEM-Bildgebung zeigt eine starke Anhaftung, die durch kovalente Hydroxyl- und Carbonylbindungen zwischen den GC-Mikrostrukturen und den oberen isolierenden und unteren Substratschichten.

Die Offenlegungsschrift US 2016/073920 A1 bezieht sich auf hybride Metall- und Kohlenstoff- oder Glaskohlenstoff-MEMS u. -ECoG-Elektroden- und Mikroelektroden-Strukturen. Es werden mikroelektromechanische Systeme offenbart, die mindestens eine Elektrode, Mikroelektrode oder eine Kombination davon umfassen, wobei die mindestens eine Elektrode ein Kohlenstoffmaterial, ein glasartiges Kohlenstoffmaterial oder eine Kombination davon umfasst. Die in Betracht gezogenen Systeme sind für µ-ECoG-Arrays geeignet. Es werden zusätzliche mikroelektromechanische Systeme offenbart, die mindestens eine Elektrode, Mikroelektrode oder eine Kombination davon umfassen, wobei die mindestens eine Elektrode ein Kohlenstoffmaterial, ein glasartiges Kohlenstoffmaterial oder eine Kombination davon umfasst; mindestens ein Substrat, eine Oberfläche, eine Schicht oder eine Kombination davon, wobei die mindestens eine Elektrode, Mikroelektrode oder Kombination davon auf dem mindestens einen Substrat, der Oberfläche, der Schicht oder einer Kombination davon angeordnet, damit gekoppelt oder anderweitig darauf geschichtet ist; und mindestens ein Bump-Pad, wobei die mindestens eine Elektrode, Mikroelektrode oder Kombination davon mit dem mindestens einen Bump-Pad über mindestens ein leitendes Metall gekoppelt ist. Ein Verfahren zur Herstellung eines mikroelektromechanischen Systems umfasst das Aufbringen eines Polymervorläufers, eines kohlenstoffhaltigen Materials oder einer Kombination davon auf eine Oberfläche, ein Substrat, mindestens eine Schicht oder eine Kombination davon; und das Erhitzen oder Pyrolysieren des Polymervorläufers, eines kohlenstoffhaltigen Materials oder einer Kombination davon, um ein glasartiges Kohlenstoffmaterial zu bilden.

In der Offenlegungsschrift WO 2019/046631 A1 wird eine Sondenvorrichtung offenbart, die eine oder mehrere isolierende Schichten und eine glasartige Kohlenstoffschicht enthält. Die glasartige Kohlenstoffschicht enthält einen oder mehrere Kanäle. Jeder Kanal weist eine Mikrostruktur auf, die einen Elektrodenbereich, einen Verbindungsbereich und einen Bump-Pad-Bereich umfassen kann. Der Elektrodenbereich kann in Kontakt mit einem menschlichen oder tierischen Patienten oder einer Versuchsperson gebracht und zur Erfassung oder Abgabe von Signalen bei Anwendungen wie Elektrokortikographie (EKoG), Elektromyographie (EMG) und Nervenstimulation verwendet werden. Ein Verfahren zur Herstellung einer Sonde umfasst die Ablagerung eines glasartigen Kohlenstoffvorläufers auf einem Substrat, die Strukturierung des Vorläufers mittels Fotolithografie, die Pyrolyse des Vorläufers, um die Bildung von glasartigem Kohlenstoff zu ermöglichen, und die Ablagerung einer oder mehrerer isolierender Schichten.

Bekannte Anordnungen haben jedoch mindestens eine Grenzfläche zwischen den Carbonelektroden und dem Metall, die leicht zu Ausfällen führt. Bei einer größeren Anzahl von Schnittstellen besteht das Risiko von Ausfällen an jeder dieser Grenzflächen.

Grundsätzlich besteht bei der Verwendung von Carbonmaterial das Problem, dass das Carbonmaterial inert ist und daher mit jeder Art von umgebendem Material nur schwer Bindungen eingeht. Dies ist nachteilig vor allem für die Haftung auf einem Substrat und die elektrische Verbindung zu einer metallischen Leiterbahn oder einem metallischen Kontaktflecken.

Weiterhin ist Carbonmaterial hart und spröde. Daher kann es bei Deformationen zum Bruch der Strukturen kommen, so dass sowohl die Flexibilität der Elektrode als auch die absolute Größe der realisierbaren Strukturen limitiert sind.

Sofern man Haftvermittler zwischen dem Carbonmaterial und damit verbundenen Metallen verwendet, erhöht dies die Anzahl der Grenzflächen nochmals und führt damit zu einer erhöhten Ausfallwahrscheinlichkeit.

Es besteht daher ein Bedarf an einem Verfahren zur Herstellung flexibler implantierbarer Elektrodenanordnungen, welches die Nachteile der bekannten Lösungen überwindet, so dass die gefertigten Elektrodenanordnungen sicher und zuverlässig sind, aber dennoch kostengünstig herstellbar sind. Weiterhin besteht ein Bedarf an einer derartigen flexiblen implantierbaren Elektrodenanordnung.

Diese Aufgabe wird durch den Gegenstand der unabhängigen Patentansprüche gelöst. Vorteilhafte Ausführungsformen der vorliegenden Erfindung sind Gegenstand der abhängigen Patentansprüche.

Dabei basiert die vorliegende Erfindung, wie in den Ansprüchen 1 und 5 definiert, auf der Idee, eine flexible polymerbasierte Elektrodenanordnung mit integrierten Kontaktstellen und elektrischen Anschlussmöglichkeiten einstückig aus einem Carbonfasermaterial herzustellen, indem das Carbonfasermaterial in ein elektrisch isolierendes Polymermaterial eingebettet wird. In vorteilhafter Weise ist das Carbonfasermaterial offenporig, so dass das erste und/oder zweite Polymermaterial wenigstens teilweise in die Carbonfaserschicht eindringen kann.

Insbesondere umfasst eine flexible implantierbare Elektrodenanordnung eine elektrisch isolierende Trägerstruktur, die ein erstes Polymermaterial aufweist, eine elektrisch leitfähige Schicht, die eine elektrisch leitfähige Carbonfaserschicht aufweist, wobei die elektrisch leitfähige Schicht strukturiert ist, um einstückig wenigstens eine implantierbare Elektrode, wenigstens eine damit verbundene Leiterbahn und wenigstens einen Kontaktflecken auszubilden, und eine elektrisch isolierende Deckschicht, wobei die Deckschicht ein zweites Polymermaterial aufweist und die elektrisch leitfähige Schicht wenigstens teilweise abdeckt. In vorteilhafter Weise kann auf diese Weise ein ganzes Array von Elektroden, die jeweils einstückig mit den entsprechenden Zuleitungen und Kontaktflecken ausgebildet sind, hergestellt werden.

Die Biegsamkeit der elektrisch leitfähigen Komponenten kann sichergestellt werden, indem ein hochflexibles Fasermaterial verwendet wird. Selbst wenn einzelne Fasern bei der Biegung brechen, bleibt die elektrische Leitfähigkeit aufgrund der mechanischen Einbettung der Carbonfaserschicht in dem Polymermaterial unverändert erhalten.

Für das erste und zweite Polymermaterial kommen verschiedenste Kunststoffe in Frage. Beispielsweise umfassen das erste und/oder das zweite Polymermaterial Polyimid, PI, Polyethylenterephthalat, PET, Polyethylen, PE, Polycabonat, PC, Polyvinylchlorid, PVC, Polyamid, PA, Polytetrafluorethylen, PTFE, Polymethylmetacrylat, PMMA, Polyetheretherketon, PEEK, Polysulfon, PSU, Poly(p-xylylene), Polydimethylsiloxan, PDMS, und/oder Polypropylen, PP. Dabei können die Trägerstruktur und die Deckschicht aus demselben Material oder aus unterschiedlichen Materialien hergestellt sein. Polyimid hat dabei mehrere Vorteile: Zum einen ist es im vollständig vernetzten Zustand besonders inert und chemisch stabil. Zum anderen kann es in Form einer flüssigen Vorstufe aufgeschleudert werden und weist darüber hinaus eine zweite, feste, aber noch nicht komplett ausgehärtete Vorform auf, in der z. B. die Anhaftung der Carbonfaserschicht und/oder der nächstfolgenden Polymerschicht verbessert ist. Schließlich gibt es fotostruktierbare Polyimidharzsysteme, die z. B. für die Herstellung der Deckschicht auf einfache Weise die Öffnung der Kontaktflecken ermöglichen.

In vorteilhafter Weise ermöglicht die erfindungsgemäße Verwendung des Carbonfasermaterials, eine multimodale Plattform für simultanes Aufzeichnen und Stimulieren von Signalen sowie für die Detektion chemischer Substanzen bereitzustellen.

Gemäß einer vorteilhaften Weiterbildung der vorliegenden Erfindung ist die Carbonfaserschicht aus einem pyrolysierten Polymermaterial hergestellt. Daher kann das Carbonfasermaterial wenigstens teilweise eine graphitische Struktur haben, d. h. sp²-kovalent hexagonal gebundene Kohlenstoffatome aufweisen, die in gegeneinander verwundenen und gefalteten Ebenen angeordnet sind. Dabei sind die einzelnen Ebenen nur über van der Waals-Kräfte gebunden. Beispielsweise kann die erfindungsgemäße Carbonfaserschicht aus Polyacrylnitril (PAN) hergestellt werden, indem das PAN an Luft stabilisiert wird und anschließend einer Pyrolyse unter Schutzgas unterzogen wird. Es ist aber für einen Fachmann klar, dass auch alle anderen gängigen Verfahren, bei denen eine Carbonfaserschicht mit ausreichender elektrischer Leitfähigkeit erzeugt wird, im Rahmen der vorliegenden Erfindung anwendbar sind. Beispielsweise können auch Cellulose oder Pech als Ausgangsstoffe dienen.

In besonders vorteilhafter Weise weist die Carbonfaserschicht ein Gewebe, ein Gewirk oder ein Vlies auf. Beispielsweise kann ein solches Vlies durch ein Elektrospinnverfahren hergestellt werden. Beim Elektrospinnen können Fasern mit Nanometer- bis Mikrometerdurchmessern hergestellt werden.

Vliese ultradünner Fasern kombinieren ihre verhältnismäßig große spezifische Oberfläche und makroporöse Eigenschaften, also Porengrößen mehrerer Mikrometer. Dies macht sie interessant für jegliche Anwendung, bei der sehr gute Diffusionseigenschaften innerhalb einer Matrix mit großer spezifischer Oberfläche vonnöten sind. Sie sind als zusammenhängendes Material selbsttragend und makroskopisch gut handhabbar. Das Elektrospinnverfahren beruht darauf, dass die Oberflächenspannung eines Flüssigkeitstropfens durch das Anlegen einer hohen elektrischen Spannung überwunden werden kann, und so aus dem Tropfen ein feiner Flüssigkeitsstrahl austritt. Für niedermolekulare Flüssigkeiten zerfällt dieser Strahl zu vielen sehr kleinen hoch geladenen Tröpfchen. Bei Verwendung polymerer Substanzen entstehen Fasern, die sich auf der Gegenelektrode als Vlies ablagern.

In vorteilhafter Weise durchdringt die Deckschicht die Carbonfaserschicht wenigstens teilweise. Dadurch kann eine feste Bindung zu der Carbonfaserschicht einerseits und zu der darunterliegenden Trägerstruktur anderseits erreicht werden.

Die vorliegende Erfindung bezieht sich weiterhin auf ein zugehöriges Verfahren zum Herstellen einer implantierbaren Elektrodenanordnung, wobei das Verfahren die folgenden Schritte umfasst:
Bereitstellen einer elektrisch isolierenden Trägerstruktur, die ein erstes Polymermaterial aufweist,
Aufbringen einer elektrisch leitfähigen Schicht, die eine elektrisch leitfähige Carbonfaserschicht aufweist, wobei die elektrisch leitfähige Schicht strukturiert ist, um einstückig wenigstens eine implantierbare Elektrode, wenigstens eine damit verbundene Leiterbahn und wenigstens einen Kontaktflecken auszubilden,
Aufbringen einer elektrisch isolierenden Deckschicht, wobei die Deckschicht ein zweites Polymermaterial aufweist und die elektrisch leitfähige Schicht wenigstens teilweise abdeckt.

Eine solche einstückige Anordnung, die sowohl die mindestens eine Elektrodenstruktur, wie auch die elektrischen Zuleitungen und die zur Kontaktierung erforderlichen Kontaktflecken beinhaltet, hat den Vorteil einer sehr effizienten Herstellbarkeit. Darüber hinaus sind keinerlei Übergänge oder Grenzflächen zwischen der Elektrode und der Zuleitung sowie zwischen der Zuleitung und der Kontaktierungsfläche vorhanden, so dass im Vergleich zu mehrteiligen Anordnungen die elektrischen Eigenschaften und die Langzeitstabilität wesentlich verbessert werden können.

Gemäß einer vorteilhaften Weiterbildung der vorliegenden Erfindung wird die elektrisch isolierende Trägerstruktur in Form einer nicht oder nur teilweise ausgehärteten Vorstufe des ersten Polymers auf einem Substrat bereitgestellt. Verwendet man beispielsweise Polyimid, so kommt als Vorstufe ein Polyimid-Precursor zum Einsatz, der durch einen Temperschritt oberhalb von 200 °C zunächst imidisiert wird und dann durch Temperung bei 400 °C unter Stickstoff zyklisiert wird. Die voll zyklisierte Polyimidschicht ist temperaturstabil bis nahe 500 °C. Der Polyimid-Precursor kann außerdem mit fotovernetzbaren Beimischungen versehen werden, so dass die noch nicht zyklisierte Polyimidschicht fotostrukturiert werden kann.

Gemäß einer vorteilhaften Weiterbildung des erfindungsgemäßen Verfahrens umfasst der Schritt des Aufbringens der elektrisch leitfähigen Schicht die folgenden Schritte:
Bereitstellen einer Carbonfasermatte;
Anbringen der Carbonfasermatte auf der elektrisch isolierenden Trägerstruktur;
Strukturieren der Carbonfasermatte.

Die Carbonfasermatte kann z. B. mittels eines Elektrospinnverfahrens als selbsttragende Komponente bereitgestellt werden und steht damit als preisgünstiges Halbzeug zur Verfügung. Die Herstellung der feinen Elektrodenstrukturen erfolgt dann unmittelbar auf dem Trägermaterial, so dass die Strukturen durch den Träger gestützt und vor Beschädigungen geschützt sind. Beispielsweise kann die Carbonfasermatte unter Verwendung einer Ätzmaskenschicht mittels Nassätzen oder Trockenätzen oder ohne Maske direkt mittels einer Laserablation strukturiert werden.

Insbesondere kann die Carbonfasermatte durch Pyrolyse eines Polymers, vorzugsweise durch Pyrolyse von Polyacrylnitril, PAN, hergestellt werden.

Ebenso wie die Trägerstruktur kann auch die Deckschicht in Form einer nicht oder nur teilweise ausgehärteten Vorstufe des zweiten Polymers auf die Carbonfaserschicht aufgebracht werden. Verwendet man beispielsweise Polyimid, so kommt als Vorstufe ein Polyimid-Precursor zum Einsatz, der durch einen Temperschritt oberhalb von 200 °C zunächst imidisiert wird und dann durch Temperung bei 400 °C unter Stickstoff zyklisiert wird. Die voll zyklisierte Polyimidschicht ist temperaturstabil bis nahe 500 °C. Der Polyimid-Precursor kann außerdem mit fotovernetzbaren Beimischungen versehen werden, so dass die noch nicht zyklisierte Polyimidschicht fotostrukturiert werden kann. Insbesondere für die Deckschicht ist dies vorteilhaft, weil hier Öffnungen für die Elektrodenspitzen und die Kontaktflecken vorgesehen werden müssen.

Grundsätzlich kann die Deckschicht in vorteilhafter Weise mittels eines Schleuderprozesses, einer Zerstäubung oder Sprühbeschichtung, einer Dampfabscheidung oder eines Vergussverfahrens abgeschieden werden, abhängig von dem jeweils verwendeten Material.

Beispielsweise können das erste und/oder das zweite Polymer Polyimid und/oder Polydimethylsiloxan aufweisen.

Wie bereits erwähnt, weist die Carbonfasermatte ein Gewebe, ein Gewirk oder ein Vlies auf, da auf diese Weise die vorteilhafte Porosität bei gleichzeitiger Flexibilität und Stabilität erreicht werden kann. Insbesondere umfasst die Herstellung der Carbonfasermatte einen Elektrospinnprozess.

Damit eine gute Haftung zwischen der ersten Polymerschicht, welche die Trägerstruktur bildet, und der zweiten Polymerschicht, welche die Deckschicht bildet, erreicht werden kann, kann das Verfahren weiterhin den Schritt des Aktivierens des ersten Polymermaterials mittels Sauerstoffplasma vor dem Abscheiden des zweiten Polymers umfassen.

Es werden nachfolgend die folgenden Begriffe und Definitionen verwendet.

Der Begriff "flexibel" bedeutet im Zusammenhang mit der vorliegenden Erfindung, dass eine Schicht oder ein Substrat biegsam und insbesondere in bestimmten Grenzen deformierbar ist, ohne zu brechen oder jedenfalls ohne die gewünschten elektrischen und mechanischen Eigenschaften zu verlieren.

Unter der Bezeichnung "elektrisch leitfähig" wird nachfolgend verstanden, dass ein Material in der Lage ist, elektrischen Strom zu leiten und sich zur Ausbildung von Elektroden eignet. Neben der Leitfähigkeit, die beispielsweise Metalle an den Tag legen, soll damit im Sinne der vorliegenden Erfindung auch die Leitfähigkeit eines halbleitenden Materials umfasst sein.

Unter der Bezeichnung "graphitisch" wird ein Kohlenstoffmaterial verstanden, das *sp²*-kovalent hexagonal gebundene Kohlenstoff-Atome aufweist, die feste Ebenen ausbilden, wobei die festen Ebenen zueinander in beliebiger Weise angeordnet sind, um die Carbonfasern auszubilden.

Zum besseren Verständnis der vorliegenden Erfindung wird diese anhand der in den nachfolgenden Figuren dargestellten Ausführungsbeispiele näher erläutert. Dabei werden gleiche Teile mit gleichen Bezugszeichen und gleichen Bauteilbezeichnungen versehen. Weiterhin können auch einige Merkmale oder Merkmalskombinationen aus den gezeigten und beschriebenen unterschiedlichen Ausführungsformen für sich eigenständige, erfinderische oder erfindungsgemäße Lösungen darstellen. Es zeigen:
- **Fig.** 1: eine schematische Draufsicht auf eine Elektrodenanordnung gemäß der vorliegenden Erfindung;
- **Fig. 2A-I**: eine schematische Darstellung eines Herstellungsprozesses für eine Elektrodenanordnung gemäß einer ersten Ausführungsform der vorliegenden Erfindung;
- Fig. 3A-G: eine schematische Darstellung eines Herstellungsprozesses für eine Elektrodenanordnung gemäß einer zweiten Ausführungsform der vorliegenden Erfindung.

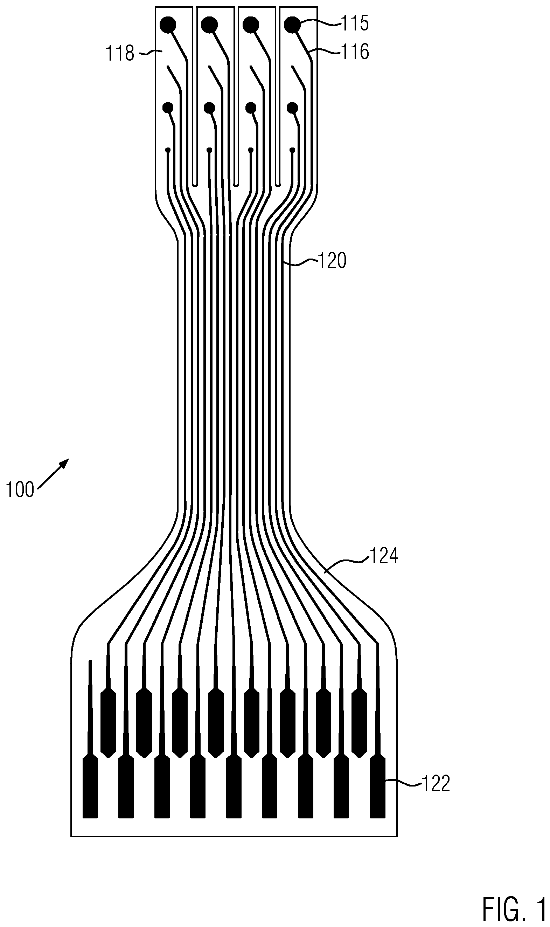

Die vorliegende Erfindung wird nachfolgend mit Bezug auf die Figuren, und dabei insbesondere zunächst mit Bezug auf die schematischen Schnittdarstellungen der Figur 1, näher erläutert. Es wird angemerkt, dass in sämtlichen Figuren die Größenverhältnisse und insbesondere die Schichtdickenverhältnisse nicht unbedingt maßstabsgetreu wiedergegeben sind.

Figur 1 zeigt in einer Draufsicht eine beispielhafte Ausführungsform einer Elektrodenanordnung 100, die ein Array von 16 einzelnen Elektroden 116 aufweist. Jeweils vier (unterschiedlich ausgestaltete) Einzelelektroden 116 sind zu einer Gruppe von Elektroden zusammengefasst, die einen Fühler 118 bilden. Über einen solchen Fühler 118 können, je nach Elektrodenform, sowohl Stimulationssignale in eine Nervenzelle eingespeist wie auch Messsignale aus der Nervenzelle abgegriffen werden.

Erfindungsgemäß sind die einzelnen Elektroden 116 jeweils einstückig mit einer Leiterbahn 120 ausgebildet. Weiterhin ist jede Leiterbahn 120 wiederum einstückig mit einer Kontaktfläche 122 (nachfolgend auch Kontaktflecken oder Kontaktpad genannt) verbunden. Dadurch erübrigen sich zwei Grenzflächen, die ansonsten für Ausfälle sorgen könnten.

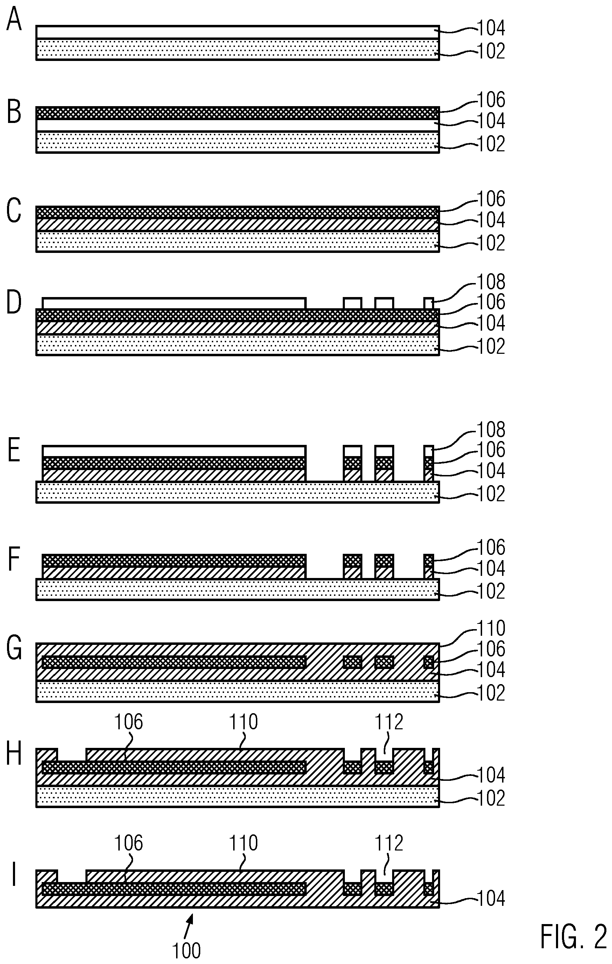

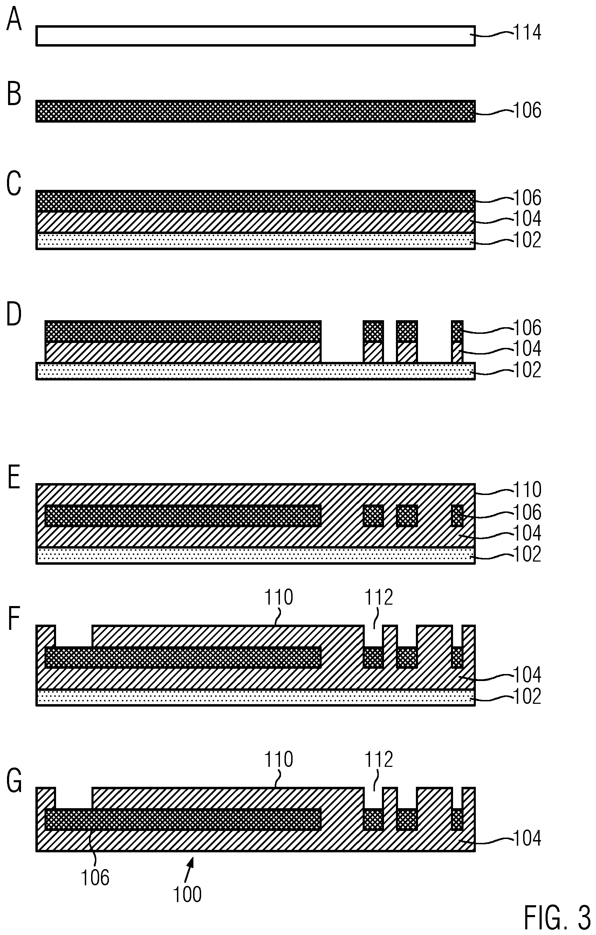

Gemäß der vorliegenden Erfindung sind alle elektrisch leitfähigen Strukturen aus einem Carbonfasermaterial hergestellt, wie dies mit Bezug auf die Figuren 2 und 3 noch im Detail erläutert wird. Zur elektrischen Isolierung sind die leitfähigen Strukturen 116, 118, 120, 122 in einem elektrisch isolierenden Polymermaterial 124 eingebettet. Dabei ist die Polymerumhüllung an den Stellen, an denen das elektrisch leitende Material zugänglich sein muss, nämlich in den aktiven Bereichen 115 der Elektroden 116 und an den Kontaktpads 122, mit entsprechenden Öffnungen versehen (siehe Figuren 2 und 3). Das Polymermaterial kann beispielsweise durch Polyimid gebildet sein.

In Experimenten konnte gezeigt werden, dass die erfindungsgemäße Elektrodenanordnung 100 stark miniaturisiert (z. B. mit kritischen Dimensionen von ca. 12,5 µm) hergestellt werden kann. Die leitfähigen Strukturen sind hoch flexibel und mechanisch stabil und es konnte eine hervorragende mechanische Verankerung zwischen der Carbonfaserschicht und dem elektrisch isolierenden Material gezeigt werden. Die Carbonfaserstrukturen zeigten auch nach 100.000 Zyklen einer Biegebeanspruchung keine messbare Abnahme der elektrischen Leitfähigkeit. Auf diese Weise stellt die vorliegende Erfindung eine komplett metallfreie und außerordentlich flexible, sowohl mechanisch wie auch elektrisch äußerst stabile Elektrodenanordnung 100 bereit.

Zusammenfassend bietet die Elektrodenanordnung gemäß der vorliegenden Erfindung die folgenden Vorteile:
- keine zusätzlichen Grenzflächen zwischen dem aktiven Bereich der Elektroden und dem Anschlussbereich an externe Komponenten,
- eine starke mechanische Einbindung der leitfähigen Strukturen in das Polymer,
- eine mechanische Flexibilität die für die strukturelle Biokompatibilität erforderlich ist,
- hohe mechanische und elektrische Stabilität des elektrisch leitfähigen Materials,
- lange Lebensdauer der Elektrode aufgrund der erhöhten Stabilität.

Die Figuren 2A bis 2I zeigen schematisch den Herstellungsprozess einer erfindungsgemäßen flexiblen implantierbaren Elektrodenanordnung 100. Fig. 2A zeigt als Ausgangsmaterial ein Substrat 102, beispielsweise ein Silizium- oder Glaswafer, auf dem die künftige Trägerstruktur 104, beispielsweise eine Polyimidschicht, aufgebracht ist. Selbstverständlich können aber auch andere Polymere, die diese erste Polymerschicht 104 ausbilden, verwendet werden.

Die Polyimidschicht kann beispielsweise mithilfe eines Aufschleuderprozesses in Form einer flüssigen Vorstufe auf dem Substrat 102 abgeschieden werden. In vorteilhafter Weise unterzieht man die aufgeschlüsselte Polyimidschicht 104 zunächst einem Trocknungsschritt, bei dem Lösemittel ausgetrieben werden, ohne jedoch die vollständige Zyklisierung herbeizuführen, bevor die Carbonfaserschicht aufgebracht wird.

Im nächsten Schritt, der in Figur 2B gezeigt ist, wird auf der Trägerstruktur 104 eine noch nicht strukturierte Carbonfaserschicht 106 abgeschieden.

Anschließend wird die Schichtfolge einem thermischen Behandlungsschritt unterzogen, bei dem die Trägerstruktur 104 in die vollständig zyklisierte Polyimidform umgewandelt wird. Dies ist durch die Schraffur angedeutet. Wie dies allgemein bekannt ist, härtet Polyimid bei ca. 400 °C aus. Selbstverständlich können auch Temperaturstufenprofile während dieses Tempervorgangs durchlaufen werden. In vorteilhafter Weise führt dieser Temperschritt dazu, dass die Carbonfasern 106 teilweise in den oberen Bereichen der Trägerstruktur 104 eingebettet sind.

Um eine Elektrodenanordnung, beispielsweise ein Array von Elektroden, und elektrische Leitungen sowie Kontaktpads auszubilden, muss die Carbonfaserschicht 106 strukturiert werden. Figur 2D illustriert schematisch, dass hierzu eine Maske 108 aufgebracht wird. Die Maske 108 lässt alle die Bereiche frei, in denen die elektrisch leitfähige Carbonfaserschicht 106 entfernt werden soll. Beispielsweise kann diese Maske 108 mithilfe einer Fotolithografie strukturiert werden, wie dies in der Halbleitertechnologie üblich ist.

Im nächsten Schritt (siehe Figur 2E) erfolgt der Materialabtrag an den nicht von der Maske 108 geschützten Bereichen durch einen Nass- oder Trockenätzschritt. Beispielsweise kann reaktives Ionenstrahlätzen (RIE) vorteilhaft eingesetzt werden. Dabei kann an den nicht von der Maske 108 abgedeckten Stellen nicht nur die Carbonfaserschicht 106, sondern auch mindestens ein Teil der Trägerstruktur 104 entfernt werden. Dies ist vorteilhaft für die spätere Anbindung einer Deckschicht. Anschließend wird die Maske 108 wieder entfernt.

Es ist aber für einen Fachmann klar, dass auch eine direkte, d. h. maskenlose, Strukturierung der Carbonfaserschicht, z. B. mittels eines Laserstrukturierungsprozesses, zur Herstellung der leitfähigen Strukturen verwendet werden kann.

Als Ergebnis des Strukturierungsprozesses liegt in jedem Fall die in Figur 2F gezeigte Anordnung vor, bei der die Elektrodenanordnung, beispielsweise ein Array von Elektroden, und elektrische Leitungen sowie Kontaktpads auf der Trägerstruktur 104 ausgebildet ist.

Im nächsten Schritt, der in Figur 2G illustriert ist, wird ganzflächig eine Deckschicht 110, die ein zweites Polymermaterial aufweist, aufgebracht. In vorteilhafter Weise verbindet sich die Deckschicht 110 mit der Trägerstruktur 104, so dass die strukturierte Carbonfaserschicht 106 vollständig von dem ersten und zweiten Polymermaterial 104, 110 umhüllt ist. Dies sorgt für eine hohe mechanische Stabilität und sichere elektrische Isolierung der Carbonfaserschicht 106.

Beispielsweise kann das zweite Polymer, das die Deckschicht 110 bildet, wiederum Polyimid sein, das in Form eines Precursor-Materials aufgeschleudert und dann in einem Temperschritt ausgehärtet wird.

Die elektrisch leitfähigen Strukturen der Carbonfaserschicht 106 müssen im Wesentlichen an zwei Schnittstellen zugänglich und mithin von der Deckschicht 110 befreit werden: Zum einen müssen die aktiven Bereiche der Elektrode mit der biologischen Umgebung in Kontakt treten können, und zum anderen müssen die Kontaktflecken elektrisch kontaktierbar sein, um die elektrischen Leiterbahnen mit weiteren elektronischen Komponenten zum Speisen und/oder Auslesen der Elektroden zu verbinden.

Fig. 2H zeigt die Anordnung, nachdem entsprechende Öffnungen 112 in die Deckschicht 110 eingebracht wurden. Für das Einbringen der Öffnungen 112 kann z. B. eine weitere Fotolithografie durchgeführt werden (die Maske ist in den Figuren nicht dargestellt), oder es kann eine direkte Strukturierung mittels Laser erfolgen. Weiterhin kann als zweites Polymermaterial direkt ein fotostrukturierbares Harz, z. B. ein fotostrukturierbares Polyimid, verwendet werden.

Im letzten Schritt wird die Elektrodenanordnung von dem sie während des Herstellungsprozesses stützenden Substrat 102 getrennt, wie dies in Fig. 2I dargestellt ist. Dies kann entweder durch Abätzen des Substrats 102 oder durch Abheben der Elektrodenanordnung 100 erfolgen.

Mit Bezug auf Fig. 3 wird nachfolgend ein abgewandeltes Herstellungsverfahren für die erfindungsgemäße Elektrodenanordnung erläutert. Für einen Fachmann ist klar, dass einzelne Merkmale der beiden Verfahren beliebig miteinander kombiniert werden können und manche der einzelnen Prozessschritte auch in unterschiedlicher Reihenfolge durchlaufen werden können. Insbesondere ist es auch möglich, die Schichtfolge von Deck- und Trägerschicht dahingehend umzukehren, dass zuerst eine Schicht mit den Kontaktöffnungen auf dem Substrat hergestellt wird, anschließend die Carbonfaserschicht aufgebracht und strukturiert wird, und schließlich die Trägerstruktur abgeschieden und gegebenenfalls ebenfalls strukturiert wird. Diese Vorgehensweise hat den Vorteil, dass beidseitige Öffnungen für Rückseitenkontaktierungen möglich sind.

Wie in Fig. 3A gezeigt, kann in einem ersten Schritt bei der Herstellung einer Elektrodenanordnung eine Polyacrylnitril (PAN)-Fasermatte 114 z. B. mittels eines Elektrospinnverfahrens hergestellt werden. Dabei wird eine 10 %-ige (Gewicht / Volumen) Lösung von PAN in Dimethylformamid (DMF) bei 10 kV und einer Polymerflussrate von 0,6 ml/h auf ein Siliziumsubstrat gesponnen. Die PAN-Fasermatte kann dann in einer sauerstoffhaltigen Atmosphäre in einer trockenen Heizkammer in 120 min bei 220 °C stabilisiert werden. Dadurch erhält man die PAN-Fasermatte 114, die in Fig. 3A gezeigt ist.

Die stabilisierte PAN-Fasermatte wird anschließend unter Stickstoffatmosphäre bei 940 °C pyrolysiert. Dabei können eine Aufheizrampe von 5 °C/min und eine Haltezeit von 60 min vorgesehen sein. Fig. 3B zeigt die entstandene Carbonfasermatte 106.

Im nächsten Schritt (Fig. 3C) wird auf einem Siliziumsubstrat 102 eine 2 µm dicke Schicht eines Polyimid-Vorläufers aufgeschleudert und bei 90° 3 Minuten lang angetrocknet. Eine zweite Polyimidschicht wird auf die erste Polyimidschicht aufgeschleudert (in der Figur nicht erkennbar), um so die Trägerstruktur 104 auszubilden. Eine Carbonfasermatte 106 wird auf die noch nicht ausgehärtete Oberfläche der Polyimidschicht aufgelegt und die in Figur 3C gezeigte Anordnung anschließend bei 90° 3 Minuten lang getrocknet (englisch: soft-curing). Die endgültige Zyklisierung erfolgt anschließend bei 450 °C.

Um die leitfähigen Strukturen in der Carbonschicht 106 auszuformen, wird im nächsten Schritt (Figur 3D) mittels eines reaktiven Ionenätzschritts (RIE) unter Verwendung von Sauerstoffplasma eine entsprechende Strukturierung vorgenommen. Dabei werden die nicht zu entfernenden Bereiche mittels einer fototechnisch strukturierten Metallisierung, die nicht in den Figuren gezeigt ist, abgedeckt und die Metallmaske anschließend wieder entfernt.

Wie in Figur 3E gezeigt, wird als Deckschicht 110 eine beispielsweise 4 µm dicke Polyimidschicht aufgeschleudert und voll zyklisiert. Optional kann vor dem Aufbringen der Deckschicht 110 die zu beschichtende Oberfläche der in Figur 3D gezeigten Anordnung mithilfe von Sauerstoffplasma (zum Beispiel 80 W bei 30 Sekunden) aktiviert werden. Dadurch wird die Haftung der Deckschicht 110 zum Untergrund verbessert. Um die äußeren Umrisse der Elektrodenanordnung zu definieren, kann nochmals ein RIE-Ätzschritt unter Verwendung einer fototechnisch hergestellten Maske durchgeführt werden.

Wie in Figur 3F gezeigt, werden außerdem die Öffnungen 112 für die aktiven Bereiche und die Kontaktflecken mithilfe eines weiteren RIE-Ätzschritts eingebracht.

Abschließend werden die einzelnen Elektrodenanordnungen 100 von dem Siliziumsubstrat 102 abgelöst (siehe Figur 3 G).

Zusammenfassend stellt die vorliegende Erfindung ein Verfahren zum Herstellen von Elektrodenanordnungen bereit, die pyrolysiertes Carbonfasermaterial zum Ausbilden der leitfähigen Strukturen, eingebettet in einem Polyimidmaterial, aufweisen. Die Carbonfaserstrukturen erwiesen sich als hoch flexibel und elektrisch wie auch mechanisch stabil. Die Haftung der einzelnen Schichten untereinander kann durch die spezielle Prozessführung auch über lange Zeiträume und in aggressiven Umgebungen sichergestellt werden. Da das Carbonfasermaterial als Fasermatte aufgebracht wird, kann es auch zur Ausbildung größerer Strukturen, wie beispielsweise Kontaktflecken verwendet werden, ohne unter der Deformation zu brechen und ohne eine zusätzliche Grenzfläche zwischen dem aktiven Elektrodenbereich und dem Anschluss an externe Geräte zu erfordern. Diese Integration resultiert in einer hohen mechanischen Stabilität und einer hohen Stabilität bei elektrischer Stimulation. Darüber hinaus führt die Verwendung von Carbonfasern dazu, dass die elektrisch leitfähigen Strukturen in das isolierende Polymermaterial eingebettet und von diesem durchdrungen sind. Weil außerdem graphitisches Carbonmaterial sehr widerstandsfähig gegenüber Korrosion ist, können Elektrodenanordnungen mit ausgezeichneter Stabilität und Langlebigkeit hergestellt werden. Daher müssen implantierte Elektroden seltener ausgetauscht werden, was für den Verwender vorteilhaft ist. Weiterhin kann das Carbonfasermaterial eingesetzt werden, um eine multimodale Plattform für simultanes Aufzeichnen, Stimulieren und Detektieren chemischer Substanzen zu ermöglichen.

### Bezugszeichenliste:

| **Bezugsziffer** | **Beschreibung** |
|---|---|
| 100 | Elektrodenanordnung |
| 102 | Substrat |
| 104 | Trägerstruktur; erste Polymerschicht |
| 106 | Carbonfaserschicht |
| 108 | Maske |
| 110 | Deckschicht |
| 112 | Öffnungen in der Deckschicht |
| 114 | PAN-Fasermatte |
| 115 | Aktiver Bereich |
| 116 | Elektrode |
| 118 | Fühler |
| 120 | Leiterbahn |
| 122 | Kontaktpad |
| 124 | Polymermaterial |

## Patentansprüche

1. Flexible implantierbare Elektrodenanordnung (100) umfassend:
eine elektrisch isolierende Trägerstruktur (104), die ein erstes Polymermaterial aufweist,
eine elektrisch leitfähige Schicht, wobei die elektrisch leitfähige Schicht strukturiert ist, um einstückig wenigstens eine implantierbare Elektrode (116), wenigstens eine damit verbundene Leiterbahn (120) und wenigstens einen Kontaktflecken (122) auszubilden, und
eine elektrisch isolierende Deckschicht (110), wobei die Deckschicht (110) ein zweites Polymermaterial aufweist und die elektrisch leitfähige Schicht (106) wenigstens teilweise abdeckt,
**dadurch gekennzeichnet, dass** die elektrisch leitfähige Schicht eine elektrisch leitfähige Carbonfaserschicht (106) aufweist, die ein Gewebe, ein Gewirk oder ein Vlies aufweist.

2. Elektrodenanordnung nach Anspruch 1, wobei das erste und/oder das zweite Polymermaterial Polyimid, PI, Polyethylenterephthalat, PET, Polyethylen, PE, Polycabonat, PC, Polyvinylchlorid, PVC, Polyamid, PA, Polytetrafluorethylen, PTFE, Polymethylmetacrylat, PMMA, Polyetheretherketon, PEEK, Polysulfon, PSU, Poly(p-xylylene), Polydimethylsiloxan, PDMS, und/oder Polypropylen, PP, umfassen.

3. Elektrodenanordnung nach Anspruch 1 oder 2, wobei die Carbonfaserschicht (106) aus einem pyrolysierten Polymermaterial hergestellt ist.

4. Elektrodenanordnung nach einem der vorhergehenden Ansprüche, wobei die Deckschicht (110) und/oder die Trägerstruktur (104) die Carbonfaserschicht wenigstens teilweise durchdringt.

5. Verfahren zum Herstellen einer implantierbaren Elektrodenanordnung (100), wobei das Verfahren die folgenden Schritte umfasst:
Bereitstellen einer elektrisch isolierenden Trägerstruktur (104), die ein erstes Polymermaterial aufweist,
Aufbringen einer elektrisch leitfähigen Schicht (106), die eine elektrisch leitfähige Carbonfaserschicht aufweist, die ein Gewebe, ein Gewirk oder ein Vlies aufweist, wobei die elektrisch leitfähige Schicht strukturiert wird, um einstückig wenigstens eine implantierbare Elektrode (116), wenigstens eine damit verbundene Leiterbahn (120) und wenigstens einen Kontaktflecken (122) auszubilden,
Aufbringen einer elektrisch isolierenden Deckschicht (110), wobei die Deckschicht ein zweites Polymermaterial aufweist und die elektrisch leitfähige Schicht wenigstens teilweise abdeckt.

6. Verfahren nach Anspruch 5, wobei die elektrisch isolierende Trägerstruktur (104) in Form einer nicht oder nur teilweise ausgehärteten Vorstufe des ersten Polymers auf einem Substrat bereitgestellt wird.

7. Verfahren nach Anspruch 5 oder 6, wobei der Schritt des Aufbringens der elektrisch leitfähigen Schicht (106) die folgenden Schritte umfasst:
Bereitstellen einer Carbonfasermatte;
Anbringen der Carbonfasermatte auf der elektrisch isolierenden Trägerstruktur;
Strukturieren der Carbonfasermatte.

8. Verfahren nach Anspruch 7, wobei die Carbonfasermatte unter Verwendung einer Ätzmaskenschicht mittels Nassätzen oder Trockenätzen oder ohne Maske direkt mittels einer Laserablation strukturiert wird.

9. Verfahren nach einem der Ansprüche 7 oder 8, wobei die Carbonfasermatte durch Pyrolyse eines Polymers, vorzugsweise durch Pyrolyse von Polyacrylnitril, PAN, hergestellt wird.

10. Verfahren nach einem der Ansprüche 5 bis 9, wobei die Deckschicht (110) in Form einer nicht oder nur teilweise ausgehärteten Vorstufe des zweiten Polymers auf die Carbonfaserschicht (106) aufgebracht wird.

11. Verfahren nach einem der Ansprüche 5 bis 10, wobei die Deckschicht (110) mittels eines Schleuderprozesses, einer Zerstäubung oder Sprühbeschichtung, einer Dampfabscheidung oder eines Vergussverfahrens abgeschieden wird.

12. Verfahren nach einem der Ansprüche 5 bis 11, wobei das erste und/oder das zweite Polymer Polyimid und/oder Polydimethylsiloxan aufweisen.

13. Verfahren nach einem der Ansprüche 5 bis 12, wobei die Herstellung der Carbonfasermatte einen Elektrospinnprozess umfasst.

14. Verfahren nach einem der Ansprüche 5 bis 13, weiterhin umfassend den Schritt des Aktivierens des ersten Polymermaterials mittels Sauerstoffplasma vor dem Abscheiden des zweiten Polymers.

## Claims

1. Flexible implantable electrode arrangement (100) comprising:
an electrically insulating carrier structure (104) comprising a first polymer material,
an electrically conductive layer, wherein said electrically conductive layer is structured in order to integrally form at least one implantable electrode (116), at least one conductor track (120) connected thereto, and at least one contact pad (122), and
an electrically insulating cover layer (110), wherein said cover layer (110) comprises a second polymer material and covers said electrically conductive layer (106) at least in part,
**characterized in that** the electrically conductive layer comprises an electrically conductive carbon fiber layer (106), which comprises a woven fabric, knitted fabric, or non-woven fabric.

2. Electrode arrangement according to claim 1, wherein said first and/or said second polymer material comprise polyimide, PI, polyethylene terephthalate, PET, polyethylene, PE, polycarbonate, PC, polyvinyl chloride, PVC, polyamide, PA, polytetrafluoroethylene, PTFE, polymethyl methacrylate, PMMA, polyether ether ketone, PEEK, polysulfone, PSU, Poly(p-xylylene), polydimethylsiloxane, PDMS, and/or polypropylene, PP.

3. Electrode arrangement according to claim 1 or 2, wherein said carbon fiber layer (106) is produced from pyrolyzed polymer material.

4. Electrode arrangement according to one of the preceding claims, wherein said cover layer (110) and/or said carrier structure (104) penetrates into said carbon fiber layer at least in part.

5. Method for the production of an implantable electrode arrangement (100), said method comprising the steps of:
providing an electrically insulating carrier structure (104) comprising a first polymer material,
applying an electrically conductive layer (106) comprising an electrically conductive carbon fiber layer, which comprises a woven fabric, knitted fabric, or non-woven fabric, wherein said electrically conductive layer is structured in order to integrally form at least one implantable electrode (116), at least one conductor track (120) connected thereto, and at least one contact pad (122),
applying an electrically insulating cover layer (110), wherein said cover layer comprises a second polymer material and covers said electrically conductive layer at least in part.

6. Method according to claim 5, wherein said electrically insulating carrier structure (104) is provided on a substrate in the form of a precursor of said first polymer that has not cured or only in part.

7. Method according to claim 5 or 6, wherein the step of applying said electrically conductive layer (106) comprises the following steps:
providing a carbon fiber mat;
attaching said carbon fiber mat to said electrically insulating carrier structure;
structuring said carbon fiber mat.

8. Method according to claim 7, wherein said carbon fiber mat is structured using an etching mask layer by way of wet etching or dry etching or without a mask directly by way of laser ablation.

9. Method according to one of the claims 7 or 8, wherein said carbon fiber mat is produced by pyrolysis of a polymer, preferably by pyrolysis of polyacrylonitrile, PAN.

10. Method according to one of the claims 5 to 9, wherein said cover layer (110) is applied onto said carbon fiber layer (106) in the form of a precursor of the first polymer that has not cured or only in part.

11. Method according to one of the claims 5 to 10, wherein said cover layer (110) is deposited in a spin-on process, by atomization, or spray coating, by vapor deposition, or in a potting process.

12. Method according to one of the claims 5 to 11, wherein said first and/or said second polymer comprise polyimide and/or polydimethylsiloxane.

13. Method according to one of the claims 5 to 12, wherein the production of said carbon fiber mat comprises an electrospinning process.

14. Method according to one of the claims 5 to 13, further comprising the step of activating said first polymer material by way of oxygen plasma prior to the deposition of said second polymer.

## Revendications

1. Agencement d'électrodes implantable flexible (100), comprenant :
une structure porteuse (104) électriquement isolante comprenant un premier matériau polymère,
une couche électriquement conductrice, la couche électriquement conductrice étant structurée afin de former d'un seul tenant au moins une électrode implantable (116),
au moins une piste conductrice (120) connectée à celle-ci et au moins un plot de contact (122), et
une couche de couverture électriquement isolante (110), la couche de couverture (110) présentant un second matériau polymère et recouvrant au moins partiellement la couche électriquement conductrice (106),
**caractérisé en ce que** la couche électriquement conductrice présente une couche de fibres de carbone électriquement conductrice (106) qui présente un tissu, un tricot ou un non-tissé.

2. Agencement d'électrodes selon la revendication 1, dans lequel le premier et/ou le second matériau polymère comprend/comprennent du polyimide PI, du polyéthylène téréphtalate PET, du polyéthylène PE, du polycabonate PC, du polychlorure de vinyle PVC, du polyamide PA, du polytétrafluoroéthylène PTFE, du polyméthacrylate de méthyle PMMA, du polyétheréthercétone PEEK, du polysulfone PSU, du poly(p-xylylène), du polydiméthylsiloxane PDMS, et/ou du polypropylène PP.

3. Agencement d'électrodes selon la revendication 1 ou 2, dans lequel la couche de fibres de carbone (106) est fabriquée à partir d'un matériau polymère pyrolysé.

4. Agencement d'électrodes selon l'une quelconque des revendications précédentes, dans lequel la couche de couverture (110) et/ou la structure porteuse (104) pénètre(nt) au moins partiellement dans la couche de fibres de carbone.

5. Procédé de fabrication d'un agencement d'électrodes implantable (100), dans lequel le procédé comprend les étapes ci-dessous consistant à :
fournir une structure porteuse (104) électriquement isolante comprenant un premier matériau polymère,
appliquer une couche électriquement conductrice (106) présentant une couche de fibres de carbone électriquement conductrice présentant un tissu, un tricot ou un non-tissé, dans laquelle la couche électriquement conductrice est structurée afin de former d'un seul tenant au moins une électrode implantable (116), au moins une piste conductrice (120) connectée à celle-ci et au moins un plot de contact (122),
appliquer une couche de couverture (110) électriquement isolante, dans lequel la couche de couverture présente un second matériau polymère et recouvre au moins partiellement la couche électriquement conductrice.

6. Procédé selon la revendication 5, dans lequel la structure porteuse (104) électriquement isolante est fournie sous la forme d'un précurseur, non ou seulement partiellement durci, du premier polymère sur un substrat.

7. Procédé selon la revendication 5 ou 6, dans lequel l'étape d'application de la couche électriquement conductrice (106) comprend les étapes ci-dessous consistant à :
fournir un mat de fibres de carbone ;
appliquer le mat de fibres de carbone sur la structure porteuse électriquement isolante ;
structurer le mat de fibres de carbone.

8. Procédé selon la revendication 7, dans lequel le mat de fibres de carbone est structuré par gravure humide ou gravure à sec en utilisant une couche formant masque de gravure, ou sans masque directement par ablation au laser.

9. Procédé selon l'une quelconque des revendications 7 ou 8, dans lequel le mat de fibres de carbone est préparé par pyrolyse d'un polymère, de manière préférée par pyrolyse de polyacrylonitrile PAN.

10. Procédé selon l'une quelconque des revendications 5 à 9, dans lequel la couche de couverture (110) est appliquée sur la couche de fibres de carbone (106) sous la forme d'un précurseur, non ou seulement partiellement durci, du second polymère.

11. Procédé selon l'une quelconque des revendications 5 à 10, dans lequel la couche de couverture (110) est déposée au moyen d'un procédé de centrifugation, d'une pulvérisation ou d'un revêtement par pulvérisation, d'un dépôt en phase vapeur ou d'un procédé d'enrobage.

12. Procédé selon l'une quelconque des revendications 5 à 11, dans lequel le premier et/ou le second polymère comprend du polyimide et/ou du polydiméthylsiloxane.

13. Procédé selon l'une quelconque des revendications 5 à 12, dans lequel la fabrication du mat de fibres de carbone comprend un procédé d'électrofilage.

14. Procédé selon l'une quelconque des revendications 5 à 13, comprenant en outre une étape consistant à activer le premier matériau polymère au moyen d'un plasma d'oxygène avant le dépôt du second polymère.
